# EUROPEAN PATENT APPLICATION

(11) **EP 2 583 664 A1**
(43) Date of publication of application: **24.04.2013**
(21) Application number: 11795587.2
(22) Date of filing: 06.06.2011
(51) Int. Cl.: A61K 8/44, A61K 8/891, A61K 8/895, A61Q 1/02, A61Q 1/12, A61Q 19/00

(54) **SKIN IMPROVING DERMO-COSMETICS**

(30) Priority: 03.06.2011 JP 2011125121; 17.06.2010 JP 2010138390
(71) Applicant: SHISEIDO COMPANY, LTD., Tokyo 104-8010 (JP)
(72) Inventor: OMURA, Takayuki, Yokohama-shi Kanagawa 224-8558 (JP); FURUKAWARA, Tomomi, Yokohama-shi Kanagawa 224-8558 (JP)
(74) Representative: Henkel, Breuer & Partner
(86) International application number: PCT/JP2011/062898
(87) International publication number: WO 2011/158679

(57) **Abstract**

Provided is a dermo-cosmetic composition to improve rough skin that: improves chapped or dry skin, particularly roughened cuticles; improves skin texture; results in smooth skin; and has excellent safety, stability and feel. The disclosed dermo-cosmetic is characterised by having: (A) one or more of D-amino acids, their salts and derivatives; and (B) a swollen material prepared by swelling one or more of non-emulsifying cross-linked silicone with an oil which is in a liquid form at room temperature.

## Description

### TECHNICAL FIELD

The present invention relates to cosmetic composition for skin. More particularly, it relates to dermo-cosmetic composition for improving skin having excellent effects of preventing or improving rough skin which are obtainable by admixing one or more of D-amino acids, their salts and derivatives and a swollen material prepared by swelling non-emulsifying cross-linked silicone with an oil which is in liquid form at room temperature.

### BACKGROUND ART

The skin roughness is a skin trouble triggered by external factors such as dryness, UV light, and irritants such as detergents and chemical substances or by internal factors such as hormonal imbalance. It is accompanied by phenomena such as roughened cuticles due to a decrease in the stratum corneum barrier function, a decrease in the stratum corneum water content, acceleration of epidermal turnover, and formation of a squamous layer (scaling). Particularly, roughened cuticles may deteriorate the adherence of cosmetics to the skin, which causes cosmetic troubles for many women.

Conventionally, in order to improve the rough skin, a method of compensating the stratum corneum barrier function using occlusive agents such as vaseline ointment and water-in-oil type emulsified preparation, a method of compensating the stratum corneum water content using moisturizers such as sorbitol, glycerin, and alkylene oxide derivatives, a method of calming skin inflammation using anti-inflammatory agents such as glycyrrhetinic acid, and a method of activating cutaneous cells using vitamin, hormone, and the like have been used (See Patent Documents 1 to 5).

However, all of the above conventional methods are not sufficient for skin water holding capacity and have low effects to improve cuticles. Further, particularly when the occlusive agents are used, a disadvantage of providing unpleasant feeling such as oily feeling and sticky feeling is caused. On the other hand, when the moisturizers are used, a large amount thereof needs to be added to increase their effects. As a result, there is a problem such that unpleasant feeling such as sticky feeling and slimy feeling is provided. Further, when extracts from animal tissues such as placenta, vitamins, hormones, and the like are used, there are problems in safety against side effects and temporal stability. Particularly, as for roughened cuticles, the situation where the cuticles are not smoothly peeled is being explained. However, there has been no appropriate measure.

### PRIOR ART PUBLICATIONS

### PATENT DOCUMENT

Patent Document 1: JP-A Hei 6-293625
Patent Document 2: JP-A Hei 7-277943
Patent Document 3: JP-A Hei 9-95432
Patent Document 4: JP-B 3660656
Patent Document 5: JP-A 2009-227645

### SUMMARY OF INVENTION

### PROBLEM TO BE SOLVED BY THE INVENTION

The present invention has been made in view of problems of the conventional art. An objective of the present invention is to provide dermo-cosmetic composition to improve rough skin that improves chapped or dry skin, particularly roughened cuticles, improves skin texture, results in smooth skin, and has excellent safety, stability, and feel.

### MEANS FOR SOLVING THE PROBLEMS

The present inventors have been dedicated to research to solve the problems. As a result, they have found out that the problems can be entirely solved by containing one or two of D-amino acids, their salts and derivatives and one or more of swollen material prepared by swelling non-emulsifying cross-linked silicone with an oil which is in a form of liquid at room temperature, and thus the present invention has been completed.

That is, the present invention is a skin improving dermo-cosmetic composition comprising:
(A) one or more of D-amino acids, their salts and derivatives; and
(B) a swollen material prepared by swelling one or more of non-emulsifying cross-linked silicone with an oil which is in a liquid form at room temperature.

### EFFECTS OF THE INVENTION

According to the present invention, there can be provided a dermo-cosmetic composition to improve rough skin that improves chapped or dry skin, particularly roughened cuticles, improves skin texture, results in smooth skin, and has excellent safety, stability, and feel.

### MODES FOR CARRYING OUT THE INVENTION

Hereinafter, the present invention will be described in detail.
The dermo-cosmetic composition of the present invention essentially contains a D-amino acid or salt or derivative thereof (ingredient (A): hereinafter also abbreviated as "D-amino acid material"). The term "D-amino acid or salt or derivatives thereof (D-amino acid material)" means D-amino acids or salt or derivatives thereof or mixtures thereof.

It is well known that amino acids can exist in L-form or D-form which are optical isomers and natural proteins are composed of L-amino acids bonded together by peptide bonds. It has been considered that except for some cases such as bacterial cell walls, only L-amino acids exist in mammals including human and only L-amino acids are used in living bodies. Conventionally, therefore, academic or industrial focus and research have been dominantly on L-amino acids.
Exceptional cases where D-amino acids are used include (1) cases where D-amino acids are used as raw materials for antibiotics to be produced by bacteria and (2) cases where food additives contain a mixture of L- and D-amino acids in order to avoid the cost required to isolate the L-amino acid from the mixture (racemic mixture) containing equal amounts of the Land D-amino acids synthesized chemically.

Recently, it has been found that even in the human body, D-aspartic acid (D-Asp), which does not occur naturally, increases with age in the ocular lens, brain, or skin, and the relationship between it and the development of cataract or Alzheimer's disease has begun to be discussed (Tadatoshi Kinouchi et al. "Proteins, Nucleic Acids, and Enzymes" (in Japanese) Vol. 50, No. 5 (2005), 453-560). Also in the skin, accumulation of D-Asp has been found to be caused by aging or ultraviolet irradiation, and it has been proposed that D-Asp be used as a molecular marker for detecting skin damage caused by aging or ultraviolet rays (Noriko Fujii, Annual Report of Cosmetology (in Japanese), No. 13 (2005). However, no examples are known in which D-amino acids are positively used as biologically active materials.
Under the above circumstances, the invention is characterized in that a D-amino acid, which has not been conventionally added to cosmetics, especially skin cosmetics, is added as an essential ingredient.

The D-amino acid material (ingredient (A)) used in the invention may be of any type as long as it is in the D-form. Preferably, the D-amino acid material itself can be effective in improving the skin. Examples include D-aspartic acid, which can have an antioxidant effect and a collagen production promoting effect, D-alanine, which can have a laminin 332 production promoting effect and a collagen production promoting effect, D-glutamic acid, which can have a barrier recovery function, a wrinkle formation reducing effect, and a skin roughness reducing effect, D-serine, which can have an ultraviolet damage reducing effect, D-hydroxyproline, which can have a laminin 332 production promoting effect, D-cysteine, which can have an ultraviolet damage reducing effect, D-methionine and D-proline, which can have an ultraviolet damage reducing effect, and D-hydroxyproline, which can have a melanin production inhibiting effect.

The D-amino acid material used in the invention may be a synthetic product or a commercially available product.
For example, a known method for producing a D-amino acid includes allowing bacterial D-aminoacylase to act on an acylated amino acid to obtain a D-amino acid (see JP-A Hei 11-113592).
The content of the D-amino acid material in the skin cosmetic composition of the invention is preferably from 0.1 to 5.0% by mass based on the total amount of the cosmetic composition.

Subsequently, the non-emulsifying cross-linked silicone as the ingredient (B) used in the present invention will be described in detail. The non-emulsifying cross-linked silicone used in the present invention is a cross-linked silicone in which some of silicone chains are cross-linked and does not have its own ability to emulsify oil and water (non-emulsifying). A cross-linked silicone is verified as "non-emulsifying" when a composition containing water, oil, and the cross-linked silicone is stirred at a high speed using, for example, a homomixer, and as a result, emulsification does not occur or emulsification occurs but the size of the emulsified particles is large such as 50 µm or more, and the emulsified state does not last when allowed to stand for a while.

Examples of the non-emulsifying cross-linked silicone used in the present invention include a cross polymer derived from a reaction between methyl hydrogen polysiloxane and methylvinyl polysiloxane (hereafter "dimethicone/vinyldimethicone cross polymer"), a cross polymer derived from a reaction between partial long chain alkylated or partial phenylated methyl hydrogen polysiloxane and methylvinyl polysiloxane (hereafter "vinyldimethicone/alkyldimethicone cross polymer"), and a cross polymer derived from a reaction between methyl hydrogen polysiloxane and alkene (hereafter "dimethicone cross polymer"). In the present invention, it is preferable to use one or more types of cross polymers selected from the group consisting of the three types of cross polymers.

For the methylvinyl polysiloxane consisting of the cross-linked silicone, those that have at least two vinyl groups in the molecule are preferably used in the present invention to effectively produce cross polymers. In actual production of the cross polymers, methylvinyl polysiloxane having one vinyl group in the molecule is commonly used and this plays a role in controlling the cross-linking ratio of the cross-polymer.

The number of carbons in the long chain alkyl group in the partially long-chain-alkylated methyl hydrogen polysiloxane can be set at will. In the present invention, a partially long-chain-alkylated methyl hydrogen polysiloxane having an alkyl group of 10 to 14 carbon atoms, in particular one having a lauryl group of 12 carbon atoms is preferably used.
On the other hand, partially phenylated methyl hydrogen polysiloxane is a compound in which some of methyl groups of methyl hydrogen polysiloxane are substituted with phenyl groups.
For alkene consisting of the dimethicone cross polymer, those having at least two vinyl groups in the molecule are preferably used in the present invention to effectively produce cross polymers. In actual production of the cross polymers, alkene having one vinyl group in the molecule is commonly used and this plays a role in controlling the cross-linking ratio of the cross-polymer.

Note that the dimethicone/vinyldimethicone cross polymer corresponds to INCI designation "(dimethicone/vinyldimethicone) cross polymer" or "polysilicone-11". Dimethicone cross polymer corresponds to INCI designation "dimethicone cross polymer". Of vinyldimethicone/alkyldimethicone cross polymers, a cross polymer derived from a reaction between laurylated methyl hydrogen polysiloxane and methyl vinyl polysiloxane corresponds to INCI designation "(vinyldimethicone/lauryldimethicone) cross polymer", and a cross polymer derived from a reaction between phenylated methyl hydrogen polysiloxane and methylvinyl polysiloxane corresponds to INCI designation (dimethicone/phenylvinyldimethicone) cross polymer. Further, a cross polymer derived from a reaction between branched-silicone containing partial long chain alkylated methyl hydrogen polysiloxane and methylvinyl polysiloxane corresponds to INCI designation "laurylpolydimethylsiloxyethyldimethicone/bis-vinyldimethic one cross polymer".

The content of the non-emulsifying cross-linked silicone (actual content) in the dermo-cosmetic composition of the present invention is preferably from 0.1 to 5.0% by mass based on the total weight of the dermo-cosmetic composition. If the content is less than 0.5% by mass, the effects of the present invention are hard to obtain. On the other hand, adding more than 5.0% by mass may not increase the effect and stickiness may be caused.

As for the dermo-cosmetic composition of the present invention, the non-emulsifying cross-linked silicone (ingredient (B)) is preferably added in a state of swollen material with liquid oil (gel composition). When it is added in such a form, it is possible to stably prepare dermo-cosmetic composition having excellent effects in improving skin quality.

As the liquid oil forming the swollen material, for example, a liquid oil having a low viscosity of 100 mPa·s or less at ambient temperature is particularly preferred. Although the lower limit of viscosity of the preferable liquid oil is not limited, a liquid oil having a viscosity of 1 mPa·s or more is preferably used. Specific examples thereof include cyclomethicone, methyltrimethicone, dimethicone, and isododecane having a viscosity within the above-described range.

In the swollen material of non-emulsifying cross-linked silicone with liquid oil, the mixing ratio of the non-emulsifying cross-linked silicone and the liquid oil is preferably 5-40:95-60 by a mass ratio.
The swollen material of non-emulsifying cross-linked silicone with liquid oil may be prepared by mixing the non-emulsifying cross-linked silicone with an appropriate liquid oil may be used, and a commercially available product may also be used. Examples of the commercially available product include the following.

Examples of a swollen materials of INCI designation dimethicone/vinyldimethicone cross polymer or polysilicone-11 include KSG-15 (a mixture of (dimethicone/vinyldimethicone) cross polymer and cyclopentasiloxane, approximately 5% of which is cross-linked (actual content)), KSG-16 (a mixture of (dimethicone/vinyldimethicone) cross polymer and dimethicone 6 mPa·s, approximately 25% of which is cross-linked (actual content)), KSG-1610 (a mixture of dimethicone/vinyldimethicone) cross polymer and methyltrimethicone, approximately 17.5% of which is cross-linked (actual content)) (these are manufactured by Shin-Etsu Chemical Co., Ltd.), GRANSIL GCM (a mixture of polysilicone-11 and octamethylcyclotetrasiloxane, approximately 6% of which is cross-linked (actual content)), GRANSIL GCM-5 (a mixture of polysilicone-11 and decamethylcyclopentasiloxane, approximately 6% of which is cross-linked (actual content)), GRANSIL IDS (a mixture of polysilicone-11 and isodecane, approximately 7% of which is cross-linked (actual content)), GRANSIL DMG-6 (a mixture of polysilicone-11 and dimethicone 6 mPa·s, approximately 18% of which is cross-linked (actual content)), GRANSIL DMG-20 (a mixture of polysilicone-11 and dimethicone 20 mPa·s, approximately 25% of which is cross-linked (actual content)), GRANSIL DMG-50 (a mixture of polysilicone-11 and dimethicone 50 mPa·s, approximately 26% of which is cross-linked (actual content)), GRANSIL PM (a mixture of polysilicone-11 and phenyltrimethicone, approximately 20% of which is cross-linked (actual content)), and GRANSIL ININ (a mixture of polysilicone-11 and isononyl isononanoate, approximately 15% of which is cross-linked (actual content)) (these are manufactured by Grant Industries, Inc.).

Examples of a swollen materials of INCI designation (dimethicone/phenylvinyldimethicone) cross polymer include KSG-18 (a mixture of (dimethicone/vinyldimethicone) cross polymer and phenyltrimethicone, approximately 15% of which is cross-linked (actual content)) (manufactured by Shin-Etsu Chemical Co., Ltd.).
Examples of a swollen materials of INCI designation vinyldimethicone/lauryldimethicone cross polymer include KSG-41 (a mixture of (vinyldimethicone/lauryldimethicone) cross polymer and liquid paraffin, approximately 30% of which is cross-linked (actual content)), KSG-42 (a mixture of (vinyldimethicone/lauryldimethicone) cross polymer and light isoparaffin, approximately 25% of which is cross-linked (actual content)), KSG-43 (a mixture of (vinyldimethicone/lauryldimethicone) cross polymer and glyceryl tri-2-ethylhexanoate, approximately 30% of which is cross-linked (actual content)), and KSG-44 (a mixture of (vinyldimethicone/lauryldimethicone) cross polymer and squalane, approximately 5% of which is cross-linked (actual content)) (all of which are manufactured by Shin-Etsu Chemical Co., Ltd.).

Examples of a swollen materials of INCI designation dimethicone cross polymer include DC9040 (a mixture of dimethicone cross polymer and decamethylcyclopentasiloxane, approximately 12% of which is cross-linked (actual content)), DC9041 (a mixture of dimethicone cross polymer and dimethicone 5 mPa·s, approximately 16% of which is cross-linked (actual content)), and DC9045 (a mixture of dimethicone cross polymer and decamethylcyclopentasiloxane, approximately 12.5% of which is cross-linked (actual content)) (all of which are manufactured by Dow Corning Toray Co., Ltd.).
Examples of a swollen materials of INCI designation lauryl polydimethylsiloxyethyl dimethicone/bis-vinyldimethicone cross polymer include KSG-042Z (a mixture of laurylpolydimethylsiloxyethyl dimethicone/bis-vinyl dimethicone cross polymer and isododecane, approximately 20% of which is cross-linked (actual content)), KSG-045Z (a mixture of laurylpolydimethylsiloxyethyl dimethicone/bis-vinyldimethicone cross polymer and cyclopentasiloxane, approximately 20% of which is cross-linked (actual content)) (all of which are manufactured by Shin-Etsu Chemical Co., Ltd.).

In the dermo-cosmetic composition of the present invention, the content of the swollen material is appropriately adjusted so that the content of non-emulsifying cross-linked silicone (actual content) is from 0.1 to 5.0% by mass based on the total weight of the dermo-cosmetic composition. If the content is beyond the above range, there is a tendency that the effects of the present invention are not sufficiently obtained and problems such as stickiness at the time of use are caused.

Arbitrary ingredients which are usually used for skin preparations for external use, such as cosmetics and medicines, for example, oils (except the liquid oils as described above), surfactants, powders, coloring agents, water, alcohols, thickeners, chelating agents, silicones, antioxidants (oxidation inhibitors), ultraviolet absorbers, moisturizers, fragrances, various medicated ingredients, antiseptics, neutralizing agents, pH regulators, and the like may be appropriately added to the dermo-cosmetic composition according to the present invention within the range not preventing the effect of the invention, if necessary.

Among the arbitrary ingredients, specific examples of oils (except the liquid oil as described above) include liquid oils such as avocado oil, Camellia oil, turtle bean oil, Macadamia nuts oil, corn oil, mink oil, olive oil, Canoga oil, egg yolk oil, sesame seed oil, Persic oil, wheat germ oil, Camellia sasanqua oil, castor oil, linseed oil, safflower oil, cotton oil, evening primrose oil, perilla oil, soybean oil, peanut oil, tea seed oil, kaya oil, rice bran oil, Chinese tung tree wood oil, Japanese tung tree wood oil, jojoba oil, germ oil, triglycerine, glyceryl trioctanoate, and glyceryl triisopalmitate; solid oil/fat such as cocoa butter, coconut butter, horse fat, hardened coconut oil, palm oil, beef tallow, mutton tallow, hardened beef tallow, palm kernel oil, lard, Japan wax kernel oil, hardened oil, Japan wax, and hardened castor oil; waxes such as beeswax, candelilla wax, carnauba wax, lanolin, lanolin acetate, liquid lanolin, sugar cane wax, fatty acid isopropyl lanolin, hexyl laurate, reduced lanolin, jojoba wax, hard lanolin, polyoxyethylene (POE hereinafter), lanolin alcohol ether, POE lanolin alcohol acetate, lanolin fatty acid polyethylene glycol, and POE hydrogenated lanolin alcohol ether; carbohydrates such as liquid paraffin, ozokerite, squalene, paraffin, ceresin, squalane, Vaseline, and microcrystalline wax; ester oils such as isopropyl myristate, cetyl octoate, octyldodecil myristate, isopropyl palmitate, butyl stearate, hexyl laurate, myristyl myristate, decyloleate, hexyldecyl dimethyl octoate, cetyl lactate, myristyl lactate, lanolin acetate, isocetyl stearate, isocetyl iso-stearate, 12-hydroxy cholesteryl stearate, di-2-ethylhexylic acid ethyleneglycol, dipentaerythritol fatty acid ester, N-alkylglycol monoisostearate, neopentylglycol dicaprate, diisostearyl malate, glyceryl di-2-heptyl undecanate, tri-methylol propane tri-2-ethylhexyl acid, tri-methylol propane triisostearate, pentaerythritol tetra-2-ethylhexyl acid, glyceryl tri-2-ethyl-hexanoate, tri-methylol propane triisostearate, cetyl-2-ethylexanoate, 2-ethylhexyl-palmitate, glycerine trimyristate, glyceride tri-2-heptyl undecatoic acid, methyl ester of castor oil fatty acid, oleate oil, acetoglyceride, palmitate-2-heptyl undecyl, diisopropyl adipate, N-lauroyl-L-glutamic acid-2-octyldodecil ester, di-2-heptylundecyl adipate, di-2-ethylhexyl sebacate, myristate-2-hexyldecyl, palmitate-2-hexyldecyl, adipate-2-hexyldecyl, diisopropyl sebacate, and succinate-2-ethylhexyl, higher fatty acids such as lauric acid, myristic acid, palmitic acid, stearic acid, behenic acid, oleic acid, 12-hydroxy-stearic acid, undecylenic acid, lanolin fatty acid, isostearic acid, linolic acid, linolenic acid, and eicosapentaenoic acid; higher alcohols of straight/branched chain such as lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, octyldodecanol; silicone oils such as dimethylpolysiloxane, and methylphenylpolysiloxane; and perfluorocarbons or perfluoropolyethers such as perfluorohexane, and triperfluoro-n-butylamine.

Examples of the surfactants include fatty acid soaps such as raw material of soap, sodium laurate, and sodium palmitate; higher alkylsulfate ester salts such as sodium laurylsulfate, and potassium laurylsulfate; alkyl-ether sulfate ester salts such as POE triethanolamine laurylsulfate, and POE sodium laurylsulfate; N-acylsarcosinate such as sodium lauroyl sarcosine; higher fatty acid amidsulfonic acid such as sodium N-myristyl-N-methyl taurine, and palm oil fatty acid sodium methyltauride; phosphate ester salts such as POE stearyl ether phosphate; sulfosuccinic acid salts such as sodium monolauroyl-monoethanolamide POE sulfosuccinate, and sodium lauryl polypropylene glycol sulfosuccinate; alkyl benzene sulfonates such as sodium linear dodecyl benzene sulfonic acid, triethanolamine linear dodecyl benzene sulfonic acid; N-acyl-glutamic acid salts such as disodium N-stearoyl glutamic acid, and monosodium N-stearoyl glutamic acid; higher fatty acid ester sulfate ester salts such as sodium hardened palm oil fatty acid glycerine sulfate; sulfated oil such as turkey red oil; anionic surfactants such as POE alkyl-ether carbonate, POE alkylarylether carbonate salts, higher fatty acid ester sulfonate salts, ester secondary alcohol sulfate salts, ester higher fatty acid alkylolamidsulfate salts, sodium lauroyl monoethanolamide succinate, and sodium casein; alkyl trimethyl ammonium salts such as stearyl trimethyl ammonium chloride, lauryl trimethyl ammonium chloride; dialkyl dimethyl ammonium salts such as distearyl dimethyl ammonium chloride; alkyl-pyridinium salts such as cetyl pyridinium chloride; cationic surfactants such as alkyl quaternary ammonium salts, alkyldimethylbenzyl ammonium salts, alkylisoquinolinium salts, dialkylmoriphonium salts, POE alkylamine, alkylamine salts, polyamine fatty acid derivatives, amyl alcohol fatty acid derivatives, and benzalkonium chloride; imidazoline ampholytic surfactants such as disodium 2-cocoyl-2-imidazolinium hydroxide-1-carboxyethyloxy salts, and betaine surfactants such as amidebetaine and sulfobetaine; sorbitan fatty acid esters such as sorbitan monooleate, sorbitan monoisostearate, sorbitan monolaurate, sorbitan monopalmitate, and sorbitan trioleate, glycerine polyglycerine fatty acids such as glycerine monocottonseed oil fatty acid, glycerine monostearate, glycerine sesquioleate, and monostearate glycerine malate salts, propylene glycol fatty acid esters such as propylene glycol monostearate, lipophilic nonionic surfactants such as hardened castor oil derivatives, glycerine alkylether, and POE-methyl polysiloxane copolymers; POE sorbitan fatty acid esters such as POE sorbitan monooleate, and POE sorbitan monostearate, POE sorbit fatty acid esters such as POE sorbit monolaurate, POE sorbit monooleate, and POE sorbit monostearate, POE glycerine fatty acid esters such as POE glycerine monooleate and POE glycerine distearate, POE fatty acid esters such as POE monooleate, POE distearate, and POE monodioleate, POE alkylethers such as POE laurylether, POE oleylether, POE cholestanol ester, POE alkylphenylethers such as POE octylphenylether, and POE nonylphenylether, POE/POP alkylethers such as POE/polyoxypropylene (hereinafter, POP) monobutyl ether, POE/POP cetylether, and POE/POP glycerine ether, POE castor oil hardened castor oil derivatives such as POE castor oil, POE hardened castor oil, POE hardened castor oil monoisostearate, and POE hardened castor oil maleate, POE beeswax/lanolin derivatives such as POE sorbit beeswax, alkanolamides such as palm oil fatty acid diethanolamide and fatty acid isopropanolamide, hydrophilic nonionic surfactants such as POE propylene glycol fatty acid esters, POE fatty acid amide, POE alkylamine, saccharose fatty acid ester, and alkyletoxydimethylamine oxide.

Examples of the powder include mica, talc, kaolin, sericite, muscovite, phlogopite, synthetic mica, lepidolite, black mica, lithia mica, synthetic mica, calcium carbonate, magnesium carbonate, silicic acid anhydride (silica), aluminium silicate, barium silicate, calcium silicate, magnesium silicate, strontium silicate, aluminium oxide, barium sulfate, iron oxide red, yellow iron oxide, black iron oxide, cobalt oxide, ultramarine, Prussian blue, titan oxide, zinc oxide, titan mica (titanium oxide coated mica), fish scale foil, bismuth oxychloride, boron nitride, red 228, red 226, blue 404, polyethylene powder, methyl polymethacrylic acid powder, polyamide resin powder (nylon powder), cellulose powder, organopolysiloxane elastomer, aluminum powder, and copper powder.

Examples of alcohols include lower alcohols such as methanol, ethanol, propanol, and isopropanol; and cholesterol, sitosterol, and lanosterol.

Examples of the thickeners include vegetable macromolecular substances such as Arabic rubber, tragacanth gum, galactan, callop gum, Cyamoposisgum, carrageenan, pectin, agar, and starch (corn, wheat, potato, rice), macromolecular substances of microorganism such as dextran, and pullulan, macromolecular starches such as carboxymethyl starch, and methylhydroxypropyl starch, animal macromolecular substances such as collagen, casein, and gelatin, macromolecular substances of celluloses such as methyl cellulose, nitro cellulose, ethyl cellulose, hydroxy ethyl cellulose, sodium cellulose sulfate, hydroxy propyl cellulose, carboxy methyl cellulose, and crystal cellulose, macromolecular substances of alginate such as sodium alginate, and propylene glycol ester alginate, macromolecular substances of vinyl such as polyvinyl methyl ether, and carboxy vinyl polymer, POE macromolecules, macromolecules of POE polyoxy propylene copolymer, acrylic macromolecular substances such as sodium polyacrylate, and amide polyacrylate, water-soluble inorganic macromolecules such as polyethylene imine, cation polymer, bentonite, aluminium magnesium silicate, laponite, hectorite, and silicate anhydride.

Examples of the chelating agents include citramalic acid, agaric acid, glyceric acid, shikimic acid, hinokitiol, gallic acid, tannic acid, caffeic acid, ethylenediamine tetraacetate, ethyleneglycol diamine tetraacetate, diethylene triamine pentacetate, phytic acid, polyphosphoric acid, metaphosphoric acid, and analogues of these agents, as well as alkali metal salts thereof, and carboxylate ester.

Examples of the ultraviolet absorbers include ultraviolet absorbers of benzoic acid, such as p-aminobenzoic acid; ultraviolet absorbers of anthranilic acid such as methyl anthranilate; ultraviolet absorbers of salicylic acid such as octyl salicylate; ultraviolet absorbers of cinnamic acid such as isopropyl p-methoxy cinnamic acid and octyl p-methoxycinnamate; ultraviolet absorbers such as urocanic acid, and ethyl urocanate; ultraviolet absorbers of benzophenone such as 2-hydroxy-4-methoxybenzophenone and dihydroxy benzophenone, ultraviolet absorbers of benzotriazol, and 2-phenylbenzimidazole-5-sulfonic acid.

Examples of the moisturizers include polyethylene glycol (hereinafter, PEG), propylene glycol, dipropylene glycol, 1,3-butylene glycol, glycerin, diglycerol, xylitol, maltitol, maltose, D-mannitol, glucose, fructose, sodium chondroitin sulfate, hyaluronate sodium, sodium lactate, glucosamine, and cyclodextrin.

Examples of the medicated ingredients that may be blended include vitamins such as vitamin A oil, retinol, retinol palpitate, pyridoxine hydrochloride, benzyl nicotinate, nicotinamide, d1-α-tocopheryl nicotinate, ascorbic acid magnesium phosphate, vitamin D₂, d1-α-tocopherol, pantothenic acid, biotin; anti-inflammatory agents such as azulene and glycyrrhizin; whitening agents such as such as arbutin, 4-methoxy salicylic acid, tranexamic acid, ethyl vitamin C, ascorbic acid magnesium phosphate; hormones such as estradiol; astringents such as zinc oxide and tannic acid; tonic agents such as L-menthol and camphor; and other agents such as lysozyme chloride, pyridoxine hydrochloride, and sulfur. A variety of extracts that exhibits various medical effects can also be blended. Examples thereof include houttuynia extract, cork tree bark extract, glycyrrhiza extract, peony extract, Moutan bark extract, loofah extract, Saxifraga stolonifera extract, eucalyptus extract, clove extract, marronnier extract, Centaurea cyanus extract, seaweed extract, and thyme extract.

Examples of the antiseptics include benzoic acid, salicylic acid, p-hydroxybenzoic acid ester (methylparaben, ethylparaben, butylparaben, etc.), sorbic acid, p-chlormetacresol, hexachlorophene, benzalkonium chloride, chlorhexidine chloride, trichlorocarbanilide, light-sensitive pigments, and phenoxyethanol.

In addition to the above agents, neutralizing agents such as 2-amino-2-methyl-1-propanol, 2-amino-2-methyl-1,3-propandiol, potassium hydroxide, triethanolamine, sodium carbonate; pH regulators such as lactic acid, citric acid, glycol acid, succinic acid, tartaric acid, malic acid, sodium hydrogencarbonate, and ammonium hydrogencarbonate; antioxidants such as ascorbic acid, α-tocopherol, and carotenoid may be added.
It should be noted that the above ingredients are merely examples, and the present invention is to be considered not to be limited thereto. These ingredients can be blended in any appropriate combination, in accordance with the recipe for the form desired.

The formulation of the dermo-cosmetic composition of the present invention may come in many forms including water-soluble, soluble, emulsified, oil-based, gel, ointment, aerosol, 2 layers of water-oil, 3 layers of water-oil-powder, and the like.

### EXAMPLES

Hereinafter, the present invention will be more specifically described with reference to the examples. However, the present invention is not limited to these examples. Unless otherwise specifically noted, the unit of content is percent by mass.
First, the evaluation method used in the present invention will be described.

### (1) Effects for improving rough skin

An experiment of continuous application of cream (in the examples or the comparative examples with the formulas shown in Tables 2 to 5 below) on the cheek of female subjects in their 20s to 50s suffering from rough skin for 2 weeks was conducted with each group containing 30 individuals. The effects for improving rough skin were evaluated by subject self-report. The evaluation was performed on a scale of 1 to 4, which are remarkable effect, substantial effect, minor effect, and no effect, in accordance with the following evaluation criteria. The results were indicated by the rate showing remarkable effect, substantial effect, minor effect (efficacy rate).

### <Evaluation criteria>

Remarkable effect: there was no dryness of skin and a smooth feeling was obtained;
Substantial effect: the dryness of skin was reduced and the smooth feeling was increased;
Minor response: the dryness of skin was reduced; and
No effect: the dryness of skin did not change or worsened.

Replicas of the skin surface forms were obtained and observed with a microscope (with 17 magnifications). The rough skin state was evaluated in accordance with the criteria shown in Table 1 below.

**[Table 1]**

| Mark | Mark Criteria |
|---|---|
| 1 | Disappearance of sulcus cutis and crista cutis as well as broadly peeling of stratum corneum |
| 2 | Unclear sulcus cutis and crista cutis as well as peeling of stratum corneum |
| 3 | Observed flat sulcus cutis and crista cutis |
| 4 | Sharp sulcus cutis and crista cutis |
| 5 | Sharp and fine sulcus cutis and crista cutis |

### (2) Texture (no stickiness at the time of use)

### <Evaluation criteria>

⊙: 25 or more felt there was no stickiness;
○: 20 to 24 felt there was no stickiness;
Δ: 15 to 19 felt there was no stickiness; and
×: 14 or less felt there was no stickiness.

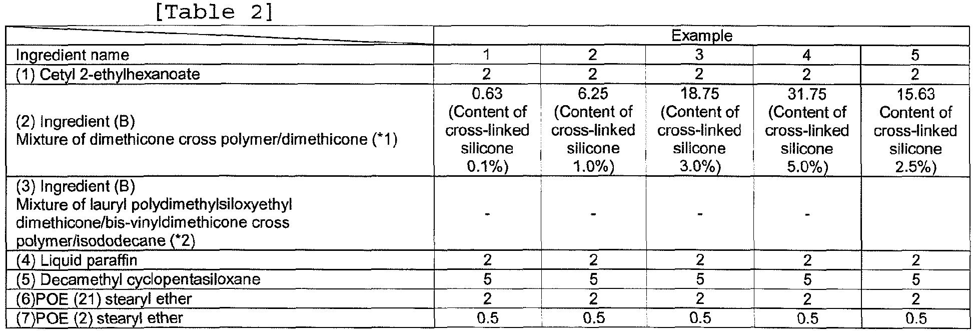

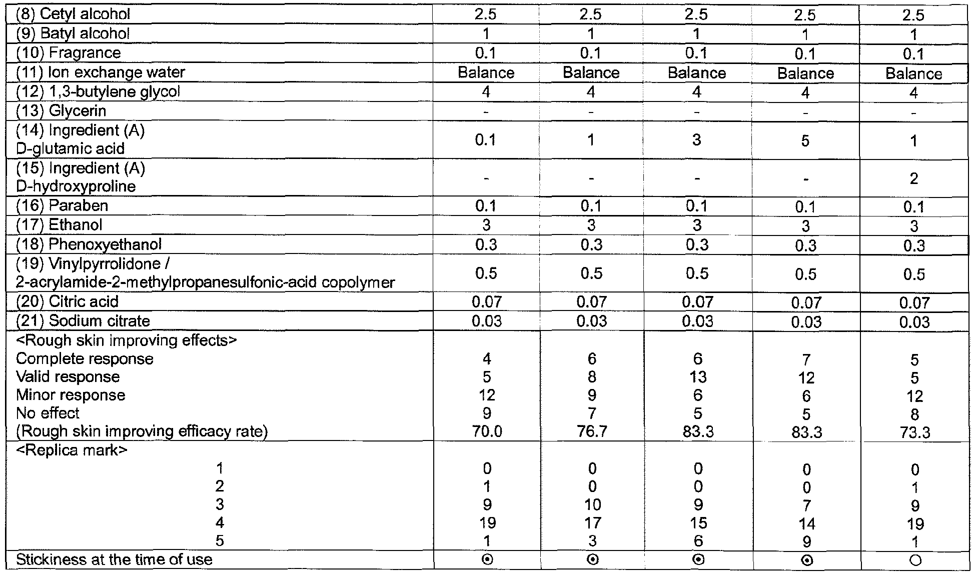

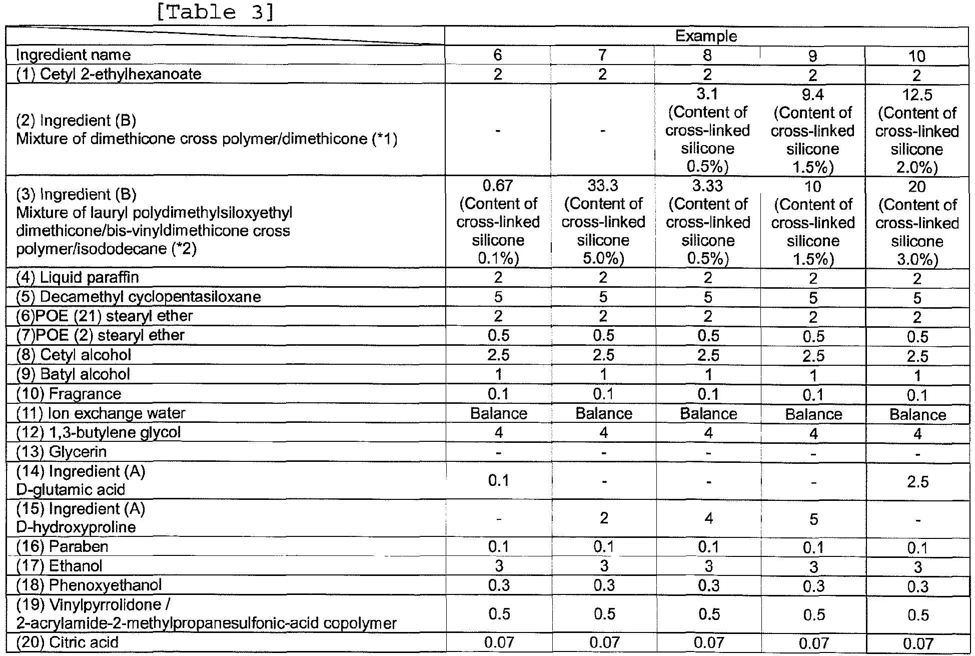

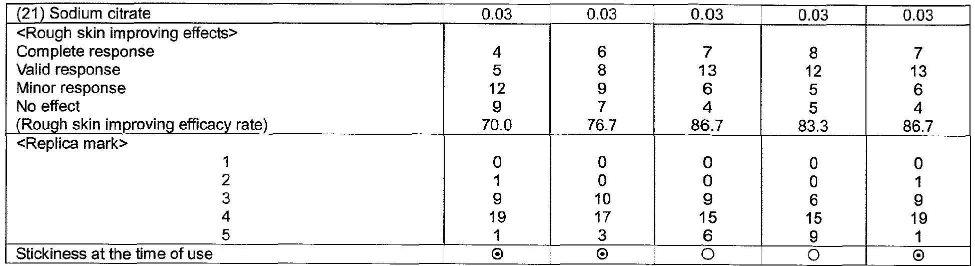

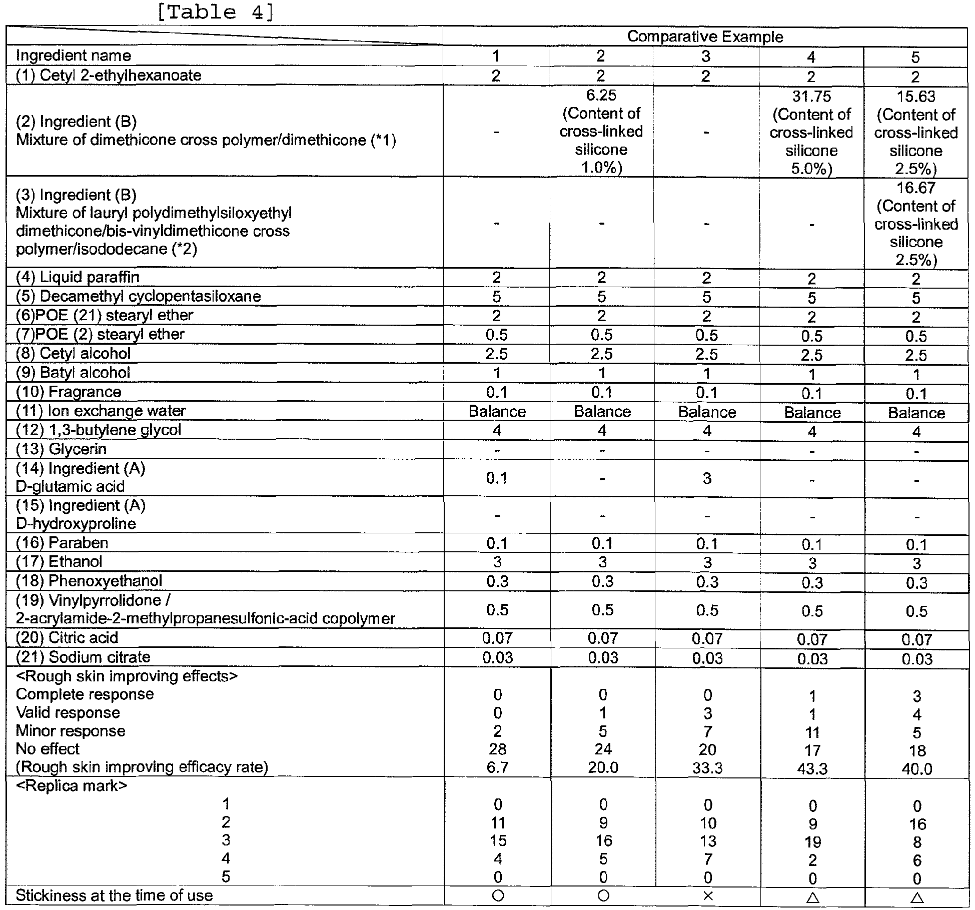

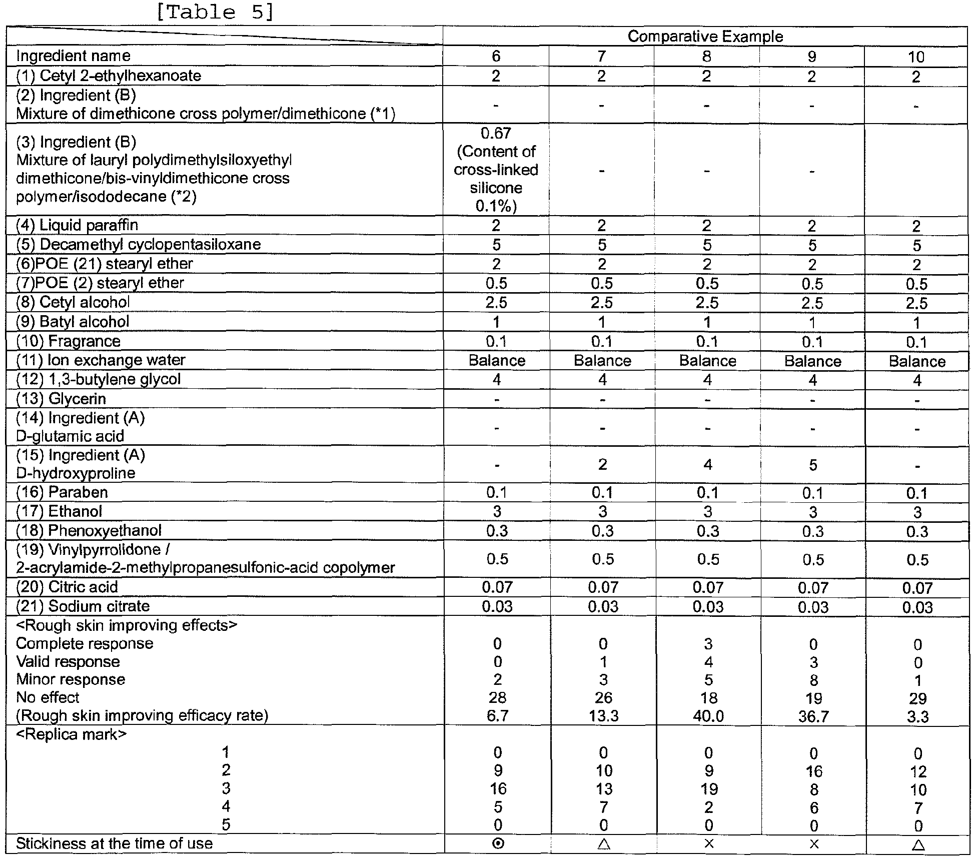

As the swollen material of non-emulsifying cross-linked silicone with liquid oil (ingredient (B)), the following materials were used:
(*1) Dimethicone cross polymer /dimethicone mixture:
   Trade name: Dow Corning 9041 Silicone ElastomerBlend (pure content of non-emulsifying cross-linked silicone 16%), manufactured by Dow Corning Toray Co., Ltd.
(*2) Lauryl polydimethylsiloxyethyl dimethicone/bis-vinyl dimethicone cross polymer/isododecane mixture:
   Trade name: KSG-042Z (pure content of non-emulsifying cross-linked silicone 15%), manufactured by Shin-Etsu Chemical Co., Ltd.

As is clear from Tables 2 to 5, Examples 1 to 10 of the present invention containing one or more of D-amino acids, derivative and salts thereof (ingredient (A)) and one or more of non-emulsifying cross-linked silicone (ingredient (B)), which are essential ingredients of the present invention, can provide dermo-cosmetic composition to improve rough skin that is excellent in texture, improves chapped or dry skin, particularly roughened cuticles, improves skin texture, results in smooth skin, and has excellent safety, stability and feel. However, it is clear that Comparative examples 1 to 10 not containing the essential ingredients of the present invention are lacking either the effects of improving skin quality or the excellent texture.

Hereinafter, other examples of the present invention will be shown.
The same experiment for the effects of the cosmetics was performed on the following example and excellent results were obtained in each case.

### Example 11. Emollient cream (O/W type)

| Ingredients | contents (% by mass) |
|---|---|
| (1) Stearyl alcohol | 2.0 |
| (2) Behenyl alcohol | 1.0 |
| (3) Hydrogenated polyisobutene | 4.0 |
| (4) Squalane | 7.0 |
| (5) Dineopentanoic acid tripropylene glycol | 2.0 |
| (6) 1,3-butylene glycol | 5.0 |
| (7) Dipropylene glycol | 3.0 |
| (8) [Ingredient (B)] | |
| Dimethicone cross polymer/dimethicone mixture (Content of cross-linked silicone 3.0%) | 25.0 |
| Trade name: Dow Corning 9045 Silicone Elastomer Blend (pure content of non-emulsifying cross-linked silicone 12%), manufactured by Dow Corning Toray Co., Ltd. | |
| (9) Polyethylene glycol 1500 | 1.0 |
| (10) Monopalm oil fatty acid polyoxyethylene (20) sorbitan Trade name: NIKKOL TL-10V, manufactured by Nikko | |
| Chemicals, Co., Ltd. | 3.0 |
| (11) Glyceryl monostearate | 2.0 |
| (12) Ethylparaben | 0.1 |
| (13) Butylparaben | 0.1 |
| (14) Tocopherol | 0.1 |
| (15) [Ingredient (A)] D-alanine | 2.0 |
| (16) Fragrance | q.s. |
| (17) Ion exchange water | balance |

### <Production process>

(6), (7), (9), and (12) to (15) were added to (17) and the resultant mixture was heated to 70°C and prepared. Then, an oil phase of (1) to (5), (8), (10), (11), and (16) was prepared at 70°C. The oil phase was added to the aqueous phase. The mixture was put in a homomixer to make emulsifying particles uniform, followed by deaeration, cooling, and filtration to yield a target emollient cream (O/W type).

### Example 12. Emulsion

| Ingredient | contents (% by mass) |
|---|---|
| (1) Dimethicone-5cs | 3.0 |
| (2) [Ingredient (B)] Polysilicone-11/decamethylcyclopentasiloxane mixture (content of non-emulsifyingcross-linked silicone 0.3%) | 5.0 |
| Trade name: GRANCIL GCM-5 (pure content of non-emulsifying cross-linked silicone 6%, manufactured by Grant Industries, Inc.) | |
| (3) Squalane | 2.0 |
| (4) Olefin oligomer | 1.0 |
| (5) Isotridecyl isononanoate | 2.0 |
| (6) PEG-20 stearate | 0.3 |
| Trade name: EMALEX 820, manufactured by Nihon Emulsion Co., Ltd. | |
| (7) Sorbitan sesquistearate | 0.1 |
| Trade name: NIKKOL SS-15V, manufactured by Nikko Chemicals, Co., Ltd. | |
| (8) Glyceryl monostearate | |
| (self-emulsifying type) | 0.3 |
| Trade name: NIKKOL MGS-ASEV, manufactured by Nikko Chemicals, Co., Ltd. | |
| (9) Fragrance | q.s. |
| (10) Dipropylene glycol | 1.0 |
| (11) 1,3-butylene glycol | 4.0 |
| (12) Glycerin | 2.0 |
| (13) Carboxyvinyl polymer | 0.1 |
| (14) Alkyl-modified carboxyvinyl polymer | 0.05 |
| (15) Potassium hydroxide | q.s. |
| (16) [Ingredient (A)] D-methionine | 3.5 |
| (17) Field horsetail extract | 0.1 |
| (18) Hamamelis virginiana extract | 0.1 |
| (19) Ethanol | 5.0 |
| (20) Phenoxyethanol | 0.3 |
| (21) Ion exchange water | balance |

### <Production process>

(10) to (21) were uniformly dissolved at 60°C (aqueous phase). Then, (1) and (3) to (9) are uniformly dissolved at 60°C, which is added to the aqueous phase, followed by emulsification with a homomixer at 60°C. Further, (2) is added to the emulsified product, which is uniformly dispersed with a disper. The dispersion liquid is subjected to deaeration, cooling, and filtration to yield a target emulsion.

### Example 13. Emollient cream (O/W type)

| Ingredient | Contents (% by mass) |
|---|---|
| (1) Behenyl alcohol | 0.1 |
| (2) Batyl alcohol | 0.5 |
| (3) Hydrogenated polyisobutene | 4.0 |
| (4) Liquid paraffin | 5.0 |
| (5) Cetyl ethylhexanoate | 1.0 |
| (6) Decamethyl cyclopentasiloxane | 15.0 |
| (7) [Ingredient (B)] (Dimethicone/phenylvinyl dimethicone) cross polymer/diphenylsiloxy phenyl trimethicone mixture (content of non-emulsifying cross-linked silicone 0.75%) | 5.0 |
| Trade name: KSG-18A (pure content of non-emulsifying cross-linked silicone 15%, manufactured by Shin-Etsu Chemical Co., Ltd.) | |
| (8) Fragrance | 0.1 |
| (9) Polyethylene glycol 20000 | 1.0 |
| (10) Ethylparaben | 0.1 |
| (11) Butylparaben | 0.1 |
| (12) Tocopherol | 0.1 |
| (13) (Dimethylacrylamide/2-acrylamide-2-acid methylpropanesulfonic copolymer | 0.2 |
| (14) [Component (A)] D-cysteine | 2.0 |
| (15) Crataegus cuneata extract | 0.1 |
| (16) Syzygium jambos leaf extract | 0.1 |
| (17) Aloe extract | 0.1 |
| (18) Sanguisorba officinalis extract | 0.1 |
| (19) Caryophylli flos extract | 0.1 |
| (20) Dokudami extract | 0.1 |
| (21) Marshmallow root extract | 0.1 |
| (22) Lithospermum erythrorhizon root extract | 0.1 |
| (23) 1,3-butylene glycol | 3.0 |
| (24) Glycerin | 3.0 |
| (25) Ion exchange water | balance |

### <Production process>

(9) to (24) were added to (25) and the resultant mixture was heated to 70°C and prepared. Then, an oil phase of (1) to (6) and (8) was prepared at 70°C. The oil phase was added to the aqueous phase, which was put in a homomixer to make emulsifying particles uniform. Further, (7) is added to the resultant product, which is uniformly dispersed with a disper. The dispersion liquid is subjected to deaeration, cooling, and filtration to yield a target emollient cream (O/W type).

### Example 14 Emollient cream (W/O type)

| Ingredient | Contents (% by mass) |
|---|---|
| (1) Microcrystalline wax | 2.0 |
| (2) Liquid paraffin | 25.0 |
| (3) Hydrogenated rape seed oil | 5.0 |
| (4) Polyglyceryl-2-dioleate | 5.0 |
| Trade name: NIKKOL DGDO, manufactured by Nikko Chemicals, Co., Ltd. | |
| (5) Butylparaben | 0.1 |
| (6) Fragrance | 0.1 |
| (7) [Ingredient (B)] (Dimethicone/vinyl dimethicone) cross polymer/methyl trimethicone mixture (content of non-emulsifying cross-linked silicone 4.0%) | 23.0 |
| Trade name: KSG-1610 | |
| (pure content of non-emulsifying cross-linked silicone 17.5%, manufactured by Shin-Etsu Chemical Co., Ltd.) | |
| (8) Sodium glutamate | 1.6 |
| (9) Serine | 0.4 |
| (10) Ion exchange water | balance |
| (11) Propylene glycol | 3.0 |
| (12) Ingredient (A) D-proline | 4.5 |
| (13) Camomile extract | 0.1 |
| (14) Sophora extract | 0.1 |

### <Production process>

Some of (10) and (8) and (9) and (4) are heated to 50°C and they are made uniform (amino acid gel). Then, the amino acid gel is uniformly dispersed in an oil phase of (1), (2), (3), (5), and (6) which has been dissolved at 70°C with a disper. The remaining (10) and (11) to (14) which have been heated to 70°C are added to the resultant dispersion liquid while sufficiently stirring the ingredients. The mixture is uniformly emulsified with a disper, followed by addition of (7). The emulsified liquid is uniformly dispersed with a disper. The dispersion liquid was subjected to deaeration, cooling, and filtration to yield a target emollient cream (W/O type).

### Example 15. General cream (O/W type) with anti-aging and whitening effect

| Ingredient | Contents (% by mass) |
|---|---|
| (1) Palmitic acid | 2.0 |
| (2) Cetyl alcohol | 1.5 |
| (3) Vaseline | 3.0 |
| (4) Squalane | 5.0 |
| (5) Triethyl hexanoyne | 2.0 |
| (6) Sorbitan oleate | 2.0 |
| Trade name: EMALEX SPO-100, manufactured by Nihon Emulsion Co., Ltd. | |
| (7) Fragrance | 0.1 |
| (8) [Ingredient (B)] (Vinyl dimethicone/lauryl dimethicone) cross polymer/isododecane mixture | 10.0 |
| (content of non-emulsifying cross-linked silicone 2.5%) | |
| Trade name: KSG-42 (pure content of non-emulsifying cross-linked silicone 25%, manufactured by Shin-Etsu Chemical Co., Ltd.) | |
| (9) Tranexamic acid | 2.0 |
| (10) (Ammonium acryloyldimethyltaurate/vinyl-pyrrolidone)copolymer | 0.1 |
| (11) Methylparaben | 0.1 |
| (12) Phenoxyethanol | 0.1 |
| (13) (Ingredient A) N-(1'-piperidine)-propionic acid | 3.0 |
| (14) Hypericum erectum extract | 0.1 |
| (15) Gambir extract | 0.1 |
| (16) Melilot extract | 0.1 |
| (17) Ion exchange water | balance |

### <Production process>

(9) to (16) were added to (17) and the mixture was heated to 70°C and prepared. Then, an oil phase of (1) to (7) was prepared at 70°C. The oil phase was added to the prepared aqueous phase. The resultant phase was put in a homomixer to make emulsifying particles uniform, followed by addition of (8). The resultant mixture was uniformly dispersed with a disper. The dispersion liquid was subjected to deaeration, cooling, and filtration to yield a target general cream with anti-aging and whitening effect (O/W type).

### Example 16. Emollient cream (W/O type)

| Ingredient | Contents (% by mass) |
|---|---|
| (1) Squalane | 15.0 |
| (2) Cetyl ethylhexanoate | 5.0 |
| (3) Isononyl isononanoate | 3.5 |
| (4) Microcrystalline wax | 1.0 |
| (5) Disteardimonium hectorite | 1.5 |
| (6) PEG-10 dimethicone | 1.0 |
| Trade name: KF-6017, manufactured by Shin-Etsu Chemical Co., Ltd. | |
| (7) Decamethyl cyclopentasiloxane | 5.0 |
| (8) [Ingredient (B)] Lauryl polydimethylsiloxyethyl | |
| dimethicone/bis-vinyl dimethicone cross polymer | 25.0 |
| (content of non-emulsifying cross-linked silicone 5.0%) | |
| Trade name: KSG-045Z (pure content of non-emulsifying cross-linked silicone 20%), manufactured by Shin-Etsu Chemical Co., Ltd. | |
| (9) Fragrance | 0.1 |
| (10) 1,3-butylene glycol | 5.0 |
| (11) Glycerin | 5.0 |
| (12) [Ingredient (A)] D-aspartic acid | 1.0 |
| (13) Ethylparaben | 0.1 |
| (14) Phenoxyethanol | 0.2 |
| (15) Ascorbic acid magnesium phosphate | 0.1 |
| (16) Wild thyme extract | 0.1 |
| (17) Camellia sinensis leaf extract | 0.1 |
| (18) Ion exchange water | balance |

### <Production process>

(1) to (7) and (9) are prepared at 70°C and uniformly dispersed and dissolved to yield an oily gel. (10) to (17) are added to (18), which is uniformly dissolved and prepared at 70°C (aqueous phase). The aqueous phase is gradually added to the prepared oily gel while sufficiently stirring. The emulsified particles are uniformly prepared with a disper, followed by addition of (8) and uniform dispersion in the disper. The dispersion liquid was subjected to deaeration, cooling, and filtration to yield a target emollient cream (W/O type).

### Example 17. Gel-like essence

| Ingredient | Contents (% by mass) |
|---|---|
| (1) Sodium polyacrylate/2-acrylamide-2-methylpropanesulfonicacid copolymer | 2.0 |
| (2) [Ingredient (B)] (Dimethicone/vinyl dimethicone) cross polymer/dimethicone mixture | 3.0 |
| (content of non-emulsifying cross-linked silicone 0.75%) | |
| Trade name: KSG-16 (pure content of non-emulsifying cross-linked silicone 25%), manufactured by Shin-Etsu Chemical Co., Ltd. | |
| (3) Dimethicone-5cs | 5.0 |
| (4) POE (20) behenyl ether | 0.5 |
| Trade name: NIKKOL BB-20, manufactured by Nikko Chemicals, Co., Ltd. | |
| (5) Ethanol | 5.0 |
| (6) Phenoxyethanol | 0.1 |
| (7) Fragrance | 0.1 |
| (8) Ion exchange water | balance |
| (9) Glycerin | 5.0 |
| (10) 1,3-butylene glycol | 3.0 |
| (11) Gardenia extract | 0.1 |
| (12) Angelica acutiloba root extract | 0.1 |
| (13) Marronnier extract | 0.1 |
| (14) Ginger extract | 0.1 |
| (15) Paeonia extract | 0.1 |
| (16) [Ingredient (A)] D-alanine | 0.3 |

### <Production process>

A mixture of (2) to (4) is added to an aqueous phase prepared by uniformly dissolving (1) and (5) to (16) and the resultant mixture is uniformly dispersed with a disper. The dispersion liquid was subjected to deaeration, cooling, and filtration to yield a target gel-like essence.
As described above, the cosmetics of Examples 11 to 17 are dermo-cosmetic composition which are stable and have excellent effects of improving skin quality.

### Example 18: W/O type foundation

| Ingredient | Contents (% by mass) |
|---|---|
| (1) Silicone-treated powder | 20.0 |
| (2) Liquid paraffin | 3.0 |
| (3) Decamethyl cyclopentasiloxane | 30.0 |
| (4) Dimethicone cross polymer/dimethicone mixture | 2.0 |
| (trade name: Dow Corning 9041 Silicone Elastomer Blend (pure content of non-emulsifying cross-linked silicone 16%)) | |
| (5) POE-modified dimethylpolysiloxane | 4.5 |
| (6) Ion exchange water | balance |
| (7) D-glutamic acid | 1.0 |
| (8) 1,3-butylene glycol | 5.0 |
| (9) Antiseptic | 0.1 |
| (10) Fragrance | q.s. |

### <Production process>

(2) to (5) were heated and dissolved at 70 to 80°C (the resultant product was used as an oil phase). Further, (7) to (9) were dissolved in (6) (the resultant product was used as an aqueous phase). (1) was added to the oil phase, which was mixed with a homomixer. (10) was added thereto and mixed. Thereafter, the aqueous phase was added to the mixture and emulsified, which was filled in a container.
The silicone-treated powder was prepared by mixing sericite (1.25 parts by weight), titanium oxide (7 parts by weight), iron oxide red (0.4 part by weight), iron oxide yellow (1.25 parts by weight), and iron oxide black (0.1 part by weight) in accordance with the methods described in JP-A Nos. 63-113081 and 63-113082, reacting the mixture with tetramethyl tetrahydrogen cyclosiloxane, and undergoing an addition reaction with tetradecene.

### Example 19: O/W type emulsified foundation

| Ingredient | Contents (% by mass) |
|---|---|
| (1) Ion exchange water | balance |
| (2) Dispersant | 0.1 |
| (3) Dipropylene glycol | 5.0 |
| (4) D-cysteine | 1.0 |
| (5) Antiseptic | 0.1 |
| (6) Isostearic acid | 2.0 |
| (7) Isohexadecyl alcohol | 7.0 |
| (8) Glyceryl monostearate | 2.0 |
| (9) Liquid paraffin | 3.0 |
| (10) Decamethyl cyclopentasiloxane | 2.0 |
| (11) GRANSIL ININ (mixture or cross-linked product of polysilicone-11 and isononyl isononanoate (actual content) approximately 15%) | 2.0 |
| (12) Sericite | 8.0 |
| (13) Titanium dioxide | 10.0 |
| (14) Iron oxide yellow | 2.0 |
| (15) Iron oxide red | 0.35 |
| (16) Iron oxide black | 0.15 |
| (17) Fragrance | q.s. |

### <Production process>

The ingredients (1) to (5) were heated to 70°C and dissolved, followed by addition of the ingredients (12) to (16) and dispersion treatment (the dispersed liquid is used as an aqueous phase/powder dispersion). The ingredients (6) to (11) and (17) in another container were heated and dissolved at 80°C. The resultant mixture was added to the aqueous phase/powder dispersion kept warm at 70°C, which was emulsified and dispersed. Thereafter, the resultant dispersion was cooled to room temperature to yield a target emulsified foundation.

### Example 20: Solid emulsified W/O foundation

| Ingredient | Contents (% by mass) |
|---|---|
| (1) Decamethyl cyclopentasiloxane | 25.0 |
| (2) Dimethylpolysiloxane | 1.0 |
| (3) Cetyl 2-ethylhexanoate | 4.0 |
| (4) Isononyl isononanoate | 7.0 |
| (5) Octocrylene | 3.0 |
| (6) Lauryl polydimethylsiloxyethyl dimethicone/bis-vinyldimethicone cross polymer/isododecane mixture | |
| Trade name: KSG-042Z | 3.0 |
| (pure content of non-emulsifying cross-linked silicone 15%) | |
| (7) Trimethoxycinnamic acidmethylbis(trimethylsiloxy)silylisopentyl | 2.0 |
| (8) 12-hydroxy stearic acid | 2.0 |
| (9) Cured castor oil | 2.0 |
| (10) Microcrystalline wax | 0.5 |
| (11) Polyoxyalkylene-modified organopolysiloxane | 2.0 |
| (12) Silicone-treated powder | 20 |
| (13) Ion exchange water | balance |
| (14) D-hydroxyproline | 1.0 |
| (15) Glycerin | 15.0 |
| (16) 1.3-butylene glycol | 2.0 |
| (17) Antiseptic | 0.1 |
| (18) Fragrance | q.s. |

### <Production process>

(1) to (11) and (18) are heated to 80°C and (12) added thereto, which is dispersed. Then, a mixture of (13) to (17) which has been preheated to 80°C is added to the dispersion liquid, which is emulsified and dispersed. Thereafter, the resultant dispersion in a liquid state is filled in a middle plate, followed by cooling to room temperature to yield a target solid emulsified foundation.
The silicone-treated powder was prepared by mixing talc (3.65 parts by weight), titanium oxide (5 parts by weight), iron oxide red (0.25 part by weight), iron oxide yellow (1 part by weight), and iron oxide black (0.1 part by weight) in accordance with the methods described in JP-A Nos. 63-113081 and 63-113082, reacting the mixture with tetramethyl tetrahydrogen cyclosiloxane, and undergoing an addition reaction with tetradecene.

### Example 21: Oil-in-water emulsion foundation

| Ingredient | Contents (% by mass) |
|---|---|
| (1) Silicone-treated titanium oxide | 8.0 |
| (2) Fatty acid-treated ultrafine particle titanium oxide | 1.0 |
| (3) Silicone-treated iron oxide (red) | 0.5 |
| (4) Silicone-treated iron oxide (yellow) | 1.5 |
| (5) Silicone-treated iron oxide (black) | 0.2 |
| (6) Polyoxyalkylene-modified organopolysiloxane | |
| | 0.5 |
| (7) Decamethyl cyclopentasiloxane | 5.0 |
| (8) Octyl p-methoxycinnamate | 4.0 |
| (9) GRANSIL ININ (mixture or cross-linked product of polysilicon-11 and isononyl isononanoate (actual content) 15%) | 1.0 |
| (10) Acrylic silicone | 4.0 |
| (11) PEG-100 hydrogenated castor oil | 2.0 |
| (12) Dynamite glycerol | 6.0 |
| (13) Xanthan gum | 0.1 |
| (14) Carboxymethyl cellulose | 0.3 |
| (15) Sodium acryloyldimethyltaurate/hydroxyethyl acrylate copolymer (content: 35 to 40% by mass)(SIMULGEL NS: manufactured by Seppic) | 1.5 |
| (16) Ethanol | 3.0 |
| (17) D-glutamic acid | 1.0 |
| (18) Antiseptic | 0.1 |
| (19) Ion exchange water | balance |

### <Production process>

(1) to (10) are mixed and dispersed with a homomixer. The resultant mixture is added to an aqueous phase prepared by dissolving (11) to (19) while stirring the mixture with the homomixer.

## Claims

1. A dermo-cosmetic composition for improving skin comprising:
(A) one or more selected from the group consisting of D-amino acids, their salts and derivatives; and
(B) a swollen material prepared by swelling one or more of non-emulsifying cross-linked silicone with an oil which is in a liquid form at room temperature.

2. The dermo-cosmetic composition for improving skin according to claim 1,
wherein the D-amino acid is selected from the group consisting of D-glutamic acid, D-alanine, D-methionine, D-hydroxyproline, D-aspartic acid, D-cysteine, D-proline, and D-serine.

3. The dermo-cosmetic composition for improving skin according to claim 1 or 2,
wherein the non-emulsifying cross-linked silicone is one or more types selected from the group consisting of dimethicone cross polymer, (dimethicone/vinyl dimethicone) cross polymer, (dimethicone/phenylvinyl dimethicone) cross polymer, (vinyl dimethicone/lauryl dimethicone) cross polymer, and lauryl polydimethylsiloxyethyl dimethicone/bis-vinyl dimethicone cross polymer.

4. The dermo-cosmetic composition for improving skin according to any one of claims 1 to 3,
wherein the content of the ingredient (A) is from 0.1 to 5.0% by mass and the content of the ingredient (B) is from 0.1 to 5.0% by mass based on the total weight of the dermo-cosmetic composition.
